# FASCICULE DE BREVET EUROPEEN

(11) **EP 0 784 962 B1**
(45) Date de publication et mention de la délivrance du brevet: **16.10.2002**
(21) Numéro de dépôt: 96810876.1
(22) Date de dépôt: 17.12.1996
(51) Int. Cl.: A61B 17/64

(54) **Réducteur de fracture**
Vorrichtung zur Reponierung von gebrochenen Knochen
Bone fracture reduction apparatus

(30) Priorité: 18.01.1996 CH 13396
(43) Date de publication de la demande: 23.07.1997
(73) Titulaire: JAQUET ORTHOPEDIE S.A., CH-1228 Plan-les-Ouates (CH)
(72) Inventeur: Campopiano, Ascanio, 80059 Torre del Greco (IT); Mata, Jacques, 1163 Etoy (CH)
(74) Mandataire: Dietlin, Henri

(56) Documents cités:
- EP-A- 0 177 270
- EP-A- 0 541 651
- US-A- 2 391 537
- US-A- 4 620 533

## Description

La présente invention est du domaine de la traumatologie et concerne plus particulièrement un appareil utilisé dans la technique de fixation externe des os fracturés.

On connaît de nombreux fixateurs externes reliés aux fragments osseux par des séries de fiches insérées dans chacun des fragments osseux. Le problème de mise en place de ce type de fixateur est de pouvoir remettre les fragments osseux dans la position d'origine exacte. On a donc déjà proposé des fixateurs permettant d'orienter les fiches, et par conséquent les fragments osseux. Par exemple dans le brevet européen EP-0.177.270 on décrit un fixateur comportant deux supports de fiches osseuses séparés par une barre télescopique, chaque support pouvant être pivoté selon trois axes orthonormaux.

Par le brevet européen No 0.541.651 de la titulaire on connaît un réducteur disposé entre deux pinces de serrage des fiches insérées dans chacun des fragments osseux, ce réducteur comportant un secteur circulaire réglable muni à chaque extrémité de moyens de rotation et de déplacement axial de l'une desdites pinces de serrage. Il permet ainsi un déplacement micrométrique des fragments osseux selon des déplacements axiaux et une rotation angulaire.

Dans le brevet US No 23 91 537, il est également possible avec le dispositif proposé de combiner un déplacement axial avec une rotation. Cependant, le dispositif décrit est plutôt destiné à effectuer une élongation ou des changements d'angle des fragments sans perturber la fonction d'élongation.

La conception de ces appareils présente toutefois un défaut majeur, car il est difficile pour le praticien de se représenter le mouvement qu'il va effectuer avec l'une des nombreuses vis ou manettes de réglage. En cherchant à améliorer le positionnement relatif des fragments osseux., on prend le risque de dérégler les résultats déjà obtenus, et ceci même avec des mécanismes complexes.

La présente invention a pour but de palier cet inconvénient en proposant un réducteur de fracture permettant de visualiser précisément le déplacement engendré en actionnant l'un des éléments de réglage, en rotation puis axialement tout en ayant six degrés de liberté clairement visualisables. Elle a pour objet un réducteur comportant un secteur circulaire réglable, des moyens de déplacements linéaires et des moyens de rotations angulaires, le secteur circulaire étant divisé en deux parties arquées glissant l'une par rapport à l'autre, chacune des parties arquées étant indirectement solidaire de fiches insérées dans chacun des fragments osseux fracturés. Il est caractérisé par deux plateaux pivotants placés dans une disposition à 90° et reliés par un bras coudé, l'un des plateaux pivotant étant relié aux moyens de déplacement linéaires reliés aux fiches d'un côté de la fracture et agencés pour permettre un déplacement linéaire graduel selon trois axes, l'autre plateau pivotant étant relié au secteur circulaire réglable solidaire des fiches de l'autre côté de la fracture, chaque plateau pivotant comprenant des moyens d'entraînement en rotation graduelle.

De préférence, un tel fixateur sera utilisé au cours de la réduction de la fracture et permettra de positionner un fixateur de conception plus simple avant d'être retiré. On peut également laisser le fixateur en place, plus spécialement si l'on envisage de procéder ultérieurement à d'autres manipulations ou corrections.

Le dessin annexé représente, à titre d'exemple non limitatif, une forme d'exécution de l'objet de l'invention.

La figure 1 est une vue en perspective d'un manipulateur selon l'invention.

La figure 2 est un schéma de principe du manipulateur de la figure 1, représentant son positionnement par rapport à un os dont les fragments sont maintenus par des fiches osseuses.

La figure 3 est une vue de dessous de la plaque de rotation horizontale du manipulateur de la figure 1, avec coupe longitudinale des composants se trouvant dans la moitié supérieure du dessin.

La figure 4 est une vue de face du double secteur arqué du manipulateur de la figure 1.

La figure 5 est une coupe longitudinale des composants du double secteur, selon la ligne brisée V-V de la figure 4.

La figure 6 est une coupe transversale selon la ligne VI-VI à la figure 4.

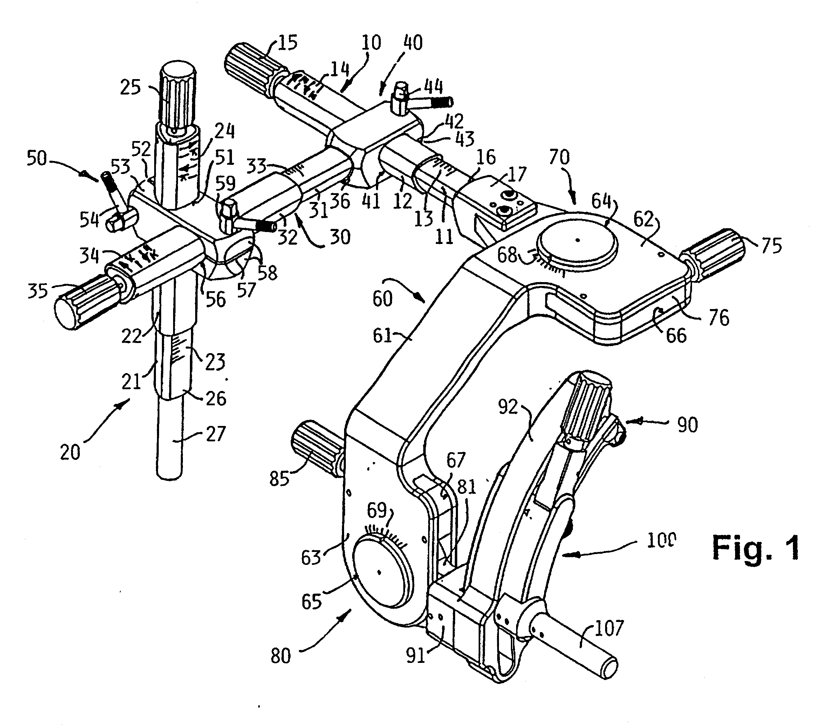

L'appareil représenté à la figure 1 comporte trois barres télescopiques 10, 20, 30, disposées respectivement selon les axes x, y et z d'un système orthonormé. Ces barres télescopiques sont reliées l'une à l'autre par des pièces de jonction 40 et 50 qui sont mobiles par rapport aux barres télescopiques, pour permettre un réglage grossier du positionnement de l'ensemble par rapport au membre fracturé, pour que le patient ne soit pas gêné.

Une extrémité de la barre 10 est reliée aux composants du manipulateur permettant d'effectuer les rotations. A cet effet, la manipulateur comporte un bras coudé 60, dont les extrémités sont constituées par deux plateaux pivotants 70 et 80 disposés dans des plans perpendiculaires, ces plateaux permettant la rotation autour des axes y et z. Dans l'exemple représenté au dessin, le plateau 70 est relié à la barre télescopique 10 et le plateau 80 est relié à un arceau 90 destiné à effectuer les rotations autour de l'axe x.

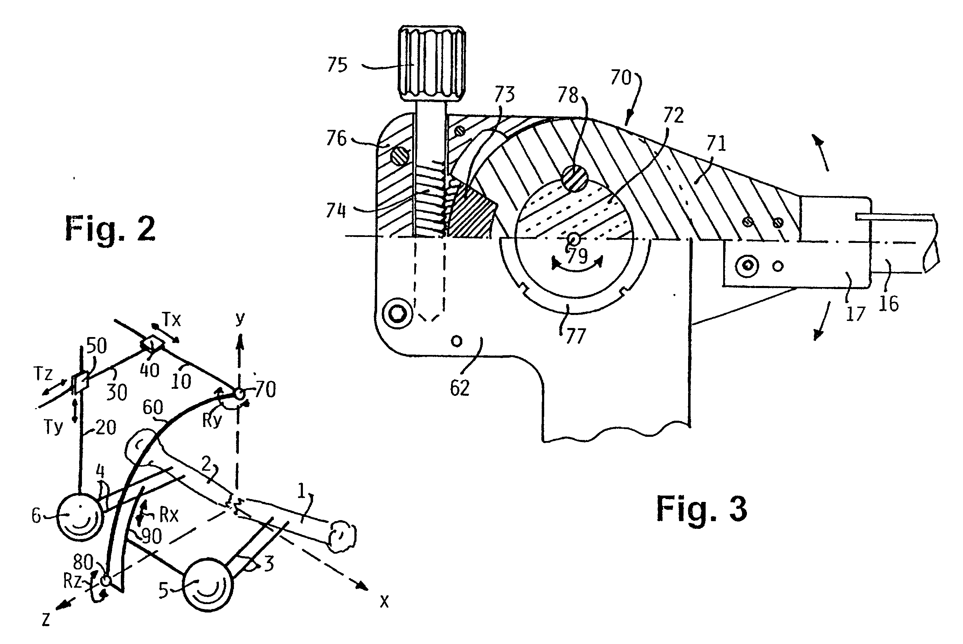

Les différents mouvements décrits ci-dessus sont détaillés dans le schéma de la figure 2 où les flèches Tx, Ty et Tz représentent respectivement les mouvements de translation selon les axes x, y et z tandis que les flèches Rx, Ry et Rz représentent les possibilités de rotation autour de ces axes.

On a également représenté à la figure 2 deux fragments 1 et 2 d'un os long, les séries de fiches osseuses 3 et 4 insérées dans les fragments 1 et 2 et maintenues dans des étaux 5 et 6, symbolisés dans ce schéma par des sphères qui sont solidaires respectivement du chariot mobile de l'arceau 90 et d'une extrémité de la barre d'attache 20.

Il est à noter à cette figure 2 que le manipulateur est disposé de telle sorte que le fragment osseux 2 est sensiblement sur l'axe x, son extrémité fracturée étant positionnée au point d'origine du système de coordonnées orthonormées x, y et z. On notera encore dans ce schéma que les barres transversales sont à gauche du plan (y,z), tandis que tous les composants permettant d'effectuer les rotations sont disposés à droite de celui-ci, de même que les fragments osseux sont répartis de part et d'autre du plan (y,z).

En revenant à la figure 1 on note que les barres télescopiques 10, 20 et 30 comportent chacune un tube intérieur 11, 21, 31 et un tube extérieur 12, 22, 32. Dans la variante représentée au dessin, ces tubes sont de section sensiblement triangulaire, pour éviter toute rotation des composants l'un par rapport à l'autre. Les tubes intérieurs 11, 21, 31 comportent une échelle millimétrique 13, 23, 33 représentant des déplacements de quelques centimètres de part et d'autre d'un point zéro. Les tubes extérieurs portent des indications fléchées 14, 24, 34, permettant de visualiser le déplacement obtenu selon le sens de rotation des boutons 15, 25, 35 disposés à l'une des extrémités des tubes extérieurs 12, 22, 32. De manière connue, ces boutons 15, 25, 35 sont solidaires d'une vis sans fin coopérant avec les tubes intérieurs des barres télescopiques.

L'extrémité libre 16 du tube intérieur 11 est fixée à une attache 17 de liaison aux composants de rotation du manipulateur. L'extrémité 26 du tube 21 est solidaire d'une barre 27 de fixation de la bride porte-fiches schématisée par la sphère 6 à la figure 2. Quant à l'extrémité libre 36 du tube 31, elle est fixée dans la pièce de jonction 40.

La pièce de jonction 40 est destinée à coulisser le long du tube extérieur 12. Elle comporte donc un dégagement 41 de forme correspondant au tube extérieur 12, le dégagement s'ouvrant sur une fente d'élasticité 42 délimitant deux mâchoires 43 destinées à être rapprochées au moyen du dispositif de serrage 44.

La pièce de jonction 50 est destinée au mouvement relatif des barres télescopiques 20 et 30. Elle comporte donc deux dégagements 51 et 56, de forme correspondant aux tubes extérieurs 22 et 32, ces dégagements s'ouvrant sur des fentes d'élasticité 52 et 57 délimitant chacune deux mâchoires 53 et 58 destinées à être rapprochées au moyen des dispositifs de serrage 54 et 59.

Comme on l'a déjà mentionné, le bras coudé 60 est centré sur la fracture. Il est réalisé en une matière transparente aux rayons X, par exemple de la fibre de carbone. Il est constitué par une partie centrale 61 entre deux plaques 62 et 63 disposées dans des plans perpendiculaires et destinées à recevoir les plateaux pivotants 70 et 80. Les plaques 62, 63 comportent chacune un passage 64, 65 de forme ronde et un dégagement 66, 67 ouvrant sur l'extérieur et destiné à recevoir les commandes de la rotation. L'angle de rotation est repérable sur les échelles 68 et 69, graduées dans l'exemple de -20° à +20°.

En se référant à la vue de détail de la figure 3 représentant le plateau pivotant 70, on y retrouve l'extrémité 16 du tube télescopique, fixée dans l'attache 17 qui est rendue solidaire d'une palette 71 solidaire d'un axe 72. La palette 71 et l'axe central 72 sont de préférence transparents aux rayons X. La palette reçoit un secteur denté 73 destiné à coopérer avec une vis sans fin 74 entraînée par un bouton 75. Ces différents composants sont maintenus dans le dégagement intérieur 66 de la plaque 62 par un corps central 76, vissé à la plaque 62 et présentant un dégagement pour le secteur denté 73 et un passage pour la vis 74. Les extrémités non filetées de la vis sans fin 74 tournent dans des paliers ou douilles, non représentés au dessin pour éviter de le surcharger. L'axe central 72 comporte, de part et d'autre de la plaque 62 une collerette 77 destinée à maintenir l'axe en place lors de son pivotement dans le passage 64 pratiqué dans la plaque 62. L'axe central 72 est d'autre part solidarisé de la palette 71 par une goupille 78, pour assurer le pivotement dans le sens des flèches lorsque l'on agit sur le bouton 75. Finalement l'axe central 72 comporte un passage central 79 permettant au praticien d'y insérer une aiguille de visée lorsqu'il centre l'appareil par rapport au fragment d'os 1 dans la position schématisée à la figure 2.

Le plateau pivotant 80 est sensiblement identique à celui qui vient d'être décrit, la seule différence résidant dans le positionnement du bouton 85 par rapport à la palette 81, et par conséquent le positionnement du secteur denté par rapport à la pièce de liaison 91 à l'arceau 90.

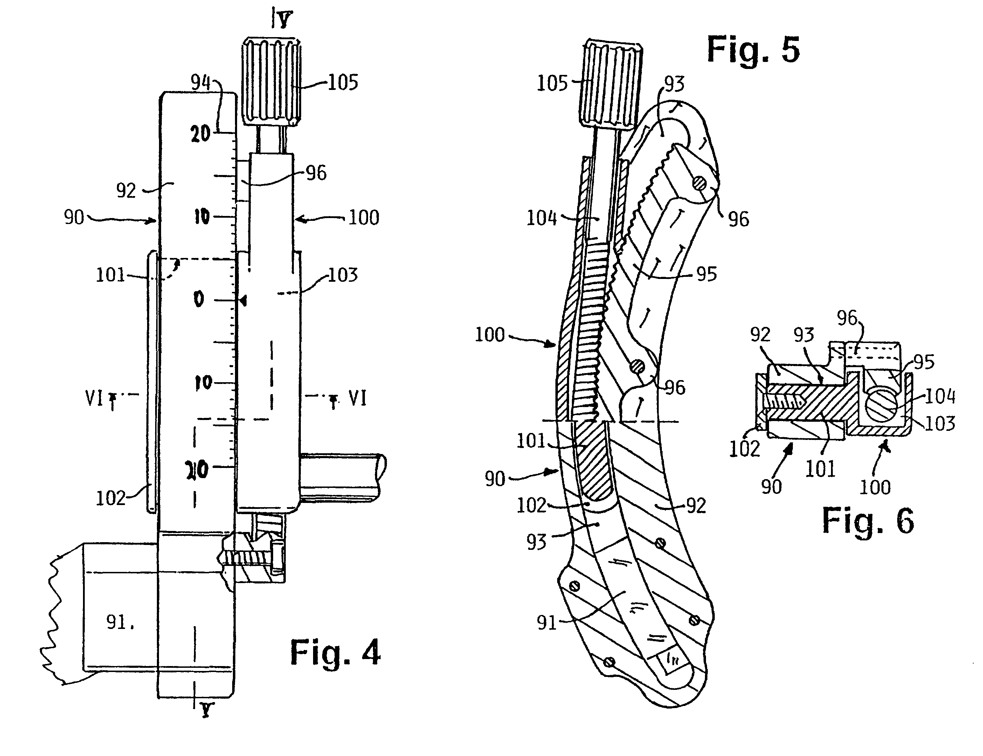

L'arceau 90 sera détaillé par rapport aux figures 4 à 6. La pièce de liaison 91 est vissée à la base de l'arceau qui est constitué par un corps 92 présentant sur toute sa hauteur une découpe arquée 93 pour le guidage d'un chariot mobile 100. Extérieurement, le corps 92 comporte des graduations 94 permettant de mesurer la rotation autour de l'axe x. Un secteur denté 95 est fixé par l'intermédiaire de trois ponts 96 au corps arqué 92.

Le chariot mobile 100 comporte une coulisse arquée 101 destinée à glisser dans la découpe 93 de l'arceau 90. Comme visible aux figures 4 et 6, la coulisse 101 reçoit, sur la gauche de l'arceau 90, un couvercle arqué 102. Sur la droite de l'arceau, la coulisse 101 se prolonge par un corps présentant une ouverture 103 destinée au passage du secteur denté 95 venant engrener avec une tige filetée 104 solidaire du bouton de réglage 105. Les parties non filetées de la vis sans fin 104 tourneront de préférence dans des paliers ou douilles non représentés au dessin. Le chariot mobile 100 entraîne une barre 107 d'attache de la bride porte-fiches schématisée par la sphère 5 à la figure 2.

Avant d'utiliser le réducteur selon l'invention, on veillera à disposer les tubes télescopiques et les éléments rotatifs dans leur position centrale, pour permettre d'effectuer des déplacements de part et d'autre de celle-ci. Dans la variante représentée au dessin, les rotations sont de -20° à +20° tandis que les déplacements linéaires sont de 3 à 4 cm de chaque côté d'un point zéro.

Pour la mise en place du réducteur selon l'invention, le praticien dispose d'abord les groupes de fiches osseuses 3 et 4 dans chaque fragment d'os 1 et 2, puis il serre ces fiches dans les porte-fiches 5 et 6 qui seront fixés respectivement sur les barres d'attache 107 et 27, tout en disposant les barres télescopiques 20 et 30 dans les positions nécessitées par chaque cas particulier, au moyen des pièces de jonction 40 et 50, qui seront alors bloquées au moyen des dispositifs de serrage 44, 54 et 59. Comme on l'a déjà mentionné, le praticien dispose le réducteur dans la position représentée à la figure 2, en disposant le fragment d'os 2 sur l'axe x, c'est-à-dire en disposant la barre 10 parallèlement au fragment osseux 2, tout en faisant coïncider les axes y et z sur le site de la fracture. Ce centrage est facilité lorsque l'on utilise une aiguille de visée dans le passage central 79 de l'axe 72, visible à la figure 3.

Pour aligner parfaitement les fragments osseux, le praticien pourra dès lors agir sur l'un des boutons 15, 25 ou 35 permettant les déplacements linéaires du fragment osseux 2 par rapport au fragment 1, tout en agissant également sur les boutons 75, 85 ou 105 assurant les déplacements en rotation. Grâce aux échelles millimétriques 13, 23 ou 33 il pourra mesurer les déplacements linéaires, tandis que les rotations seront indiquées sur les échelles 68, 69 et 94, graduées en degrés.

L'avantage de ce type de manipulateur est que, puisque chaque réglage est indépendant, le praticien peut visualiser clairement quel sera le déplacement effectué au moyen de l'un des trois boutons de déplacement linéaire ou au moyen de chacun des trois boutons de déplacements en rotation. En outre comme à chaque mouvement correspond une échelle graduée, il pourra déplacer d'une valeur précise l'un des fragments osseux par rapport à l'autre.

Comme on l'a déjà mentionné, la majorité des composants du bras coudé 60 sont transparents au rayons X de sorte que le médecin pourra vérifier aisément la position relative des fragments osseux au cours des opérations de réduction.

Lorsque les fragments osseux sont remis dans leur position originale, le praticien peut remplacer le réducteur par un fixateur externe, en fixant celui-ci sur les fiches osseuses. Selon les cas particuliers, il est également possible d'utiliser le réducteur comme fixateur externe, surtout si l'on prévoit des réglages ultérieurs.

## Revendications

1. Réducteur de fracture comportant un secteur circulaire réglable (90), des moyens de déplacement linéaires (20) et des moyens de rotation angulaires (70), le secteur circulaire réglable étant divisé en deux parties arquées glissant l'une par rapport à l'autre, chacune des parties arquées étant indirectement solidaire de fiches destinées à être insérées dans les fragments osseux de part et d'autre de la fracture, **caractérisé par** deux plateaux pivotants (70,80) placés dans une disposition à 90° et reliés par un bras coudé (60), l'un des plateaux pivotants (70) étant relié aux moyens du déplacement linéaires (10,20,30) reliés aux fiches d'un côté de la fracture et agencés pour permettre un déplacement linéaire graduel selon trois axes, l'autre plateau pivotant (80) étant relié au secteur circulaire réglable (90,100) solidaire des fiches de l'autre côté de la fracture, chaque plateau pivotant comprenant des moyens d'entraînement en rotation graduelle.

2. Réducteur selon la revendication 1, **caractérisé en ce que** le bras coudé est en une matière transparente aux rayons X.

3. Réducteur selon la revendication 1, **caractérisé en ce que** les axes des plateaux pivotants définissent un plan sur lequel est sensiblement situé la fracture et **en ce que** l'axe du secteur circulaire réglable est sensiblement perpendiculaire au plan défini par les axes des plateaux pivotants.

4. Réducteur selon la revendication 3, **caractérisé en ce que** chaque plateau pivotant (70, 80) comporte une palette (71) solidaire d'un axe (72), ledit plateau étant destiné à pivoter par rapport audit axe, la palette présentant un secteur denté (73) destiné à coopérer avec une vis sans fin (74) d'entraînement en rotation graduelle.

5. Réducteur selon la revendication 4, **caractérisé en ce que** les plateaux pivotants sont disposés dans des plans sensiblement perpendiculaires et autorisent une rotation de plus ou moins 30° par rapport à une position centrale.

6. Réducteur selon la revendication 1, **caractérisé en ce que** les moyens de déplacements linéaires comportent trois barres télescopiques (10, 20, 30) reliées entre elles par des pièces de jonction (40, 50) mobiles par rapport auxdites barres.

7. Réducteur selon la revendication 6, **caractérisé en ce que** chaque pièce de jonction (40, 50) comporte au moins un dégagement (41, 51, 56) de forme correspondant à la section d'une barre et ouvrant sur une fente d'élasticité (42, 52, 57), ainsi que des moyens de serrage (44, 54, 59) aptes à fixer la pièce de jonction sur la barre correspondante.

8. Réducteur selon la revendication 6, **caractérisé en ce que** chaque barre téléscopique (10, 20, 30) est constitué par un tube intérieur (11, 21, 31) muni d'une échelle millémètrique (13, 23, 33) et d'un tube extérieur (12, 22, 32) portant des indications fléchées (14, 24, 34) aptes à indiquer le déplacement des fragments osseux selon le sens de rotations de boutons (15, 25, 35) solidaires de vis sans fin de déplacement relatif desdits tubes.

9. Réducteur selon la revendication 8, **caractérisé en ce que** l'amplitude possible dudit déplacement est de quelques centimètres de chaque côté d'une position centrale.

10. Réducteur selon la revendication 6, **caractérisé en ce que** lesdites barres sont de section sensiblement triangulaire.

11. Réducteur selon la revendication 6, **caractérisé en ce que** lesdites barres sont disposées selon trois axes orthonormés.

12. Réducteur selon la revendication 6, **caractérisé en ce que** l'extrémité (27) d'une desdites barres constitue un support pour un étau (6) de liaison aux fiches osseuses (4, fig. 2).

13. Réducteur selon la revendication 1, **caractérisé en ce que** le secteur circulaire réglable est constitué par un arceau (90) solidaire de moyens de déplacement d'un chariot mobile (100).

14. Réducteur selon la revendication 13, **caractérisé en ce que** lesdits moyens de déplacement sont constitués par un secteur denté arqué (95) fixé à l'arceau (90) et destiné à coopérer avec une vis sans fin (104) solidaire du chariot mobile (100).

15. Réducteur selon la revendication 13, **caractérisé en ce que** l'arceau présente un dégagement arqué (93) destiné à coopérer avec une coulisse (101) solidaire du chariot mobile.

16. Réducteur selon la revendiation 13, **caractérisé en ce que** l'arceau comporte des graduations (94) donnant le déplacement angulaire du chariot mobile par rapport à l'arceau.

## Claims

1. Fracture reducer made up of an adjustable circular sector (90), means of linear movement (20) and means of angular rotation (70), the adjustable circular sector being divided into two arc-shaped parts that slide relative to each other, each of the arc-shaped parts being indirectly attached to pins designed to be inserted into the bone fragments on each side of the fracture, **characterised by** two pivoting platforms (70, 80) placed at 90° to each other and connected by a jointed arm (60); one of the pivoting platforms (70) is connected to the means of linear movement (10, 20, 30) attached to the pins in one side of the fracture and arranged so as to allow for gradual linear movement along three axes, and the other pivoting platform (80) is connected to the adjustable circular sector (90, 100) attached to the pins in the other side of the fracture. Both pivoting platforms have means of gradual rotational movement.

2. Reducer in accordance with claim 1, **characterised by** the fact that the jointed arm is made of a material pervious to X-rays.

3. Reducer in accordance with claim 1, **characterised by** the fact that the axes of the pivoting platforms define a plane on which the fracture is approximately situated, and by the fact that the axis of the adjustable circular sector is approximately perpendicular to the plane defined by the axes of the pivoting platforms.

4. Reducer in accordance with claim 3, **characterised by** the fact that each pivoting platform (70, 80) contains a paddle (71) connected to a shaft (72), the aforementioned platform being designed to pivot relative to the aforementioned shaft and the paddle presenting a toothed sector (73) designed to work in conjunction with a worm (74) for gradual rotational movement.

5. Reducer in accordance with claim 4, **characterised by** the fact that the pivoting platforms are located on approximately perpendicular planes and allow for a rotation of plus or minus 30° relative to a central position.

6. Reducer in accordance with claim 1, **characterised by** the fact that the means of linear movement are made up of three telescopic rods (10, 20, 30) connected to each other by joining pieces (40, 50) that are movable relative to the aforementioned rods.

7. Reducer in accordance with claim 6, **characterised by** the fact that each joining piece (40, 50) has at least one hole (41, 51, 56) of the same shape as the section of a rod, opening onto a flexibility slit (42, 52, 57), together with tightening devices (44, 54, 59) used to fix the joining piece to the corresponding rod.

8. Reducer in accordance with claim 6, **characterised by** the fact that each telescopic rod (10, 20, 30) is made up of an inner tube (11, 21, 31) fitted with a millimetre scale (13, 23, 33), and an outer tube (12, 22, 32) marked with arrows (14, 24, 34) used to indicate the movement of the bone fragments in accordance with the direction of rotation of the knobs (15, 25, 35) connected to worms for the relative movement of the aforementioned tubes.

9. Reducer in accordance with claim 8, **characterised by** the fact that the possible amplitude of the aforementioned movement is a few centimetres on each side of a central position.

10. Reducer in accordance with claim 6, **characterised by** the fact that the aforementioned rods have an approximately triangular section.

11. Reducer in accordance with claim 6, **characterised by** the fact that the aforementioned rods are arranged along three orthonormal axes.

12. Reducer in accordance with claim 6, **characterised by** the fact that the end (27) of one of the aforementioned rods has a mounting for a clamp (6) for connection to the bone pins (4, fig. 2).

13. Reducer in accordance with claim 1, **characterised by** the fact that the adjustable circular sector is made up of a bow (90) connected to the means of movement of a movable carriage (100).

14. Reducer in accordance with claim 13, **characterised by** the fact that the aforementioned means of movement are made up of an arc-shaped toothed sector (95) attached to the bow (90) and designed to work in conjunction with a worm (104) connected to the movable carriage (100).

15. Reducer in accordance with claim 13, **characterised by** the fact that the bow has an arc-shaped slot (93) designed to work in conjunction with a slide (101) connected to the movable carriage.

16. Reducer in accordance with claim 13, **characterised by** the fact that the bow has graduations (94) showing the angular movement of the movable carriage relative to the bow.

## Patentansprüche

1. Knochenrepositionsgerät mit einem einstellbaren Kreisbogen (90), linearen Verstellvorrichtungen (20) und angularen Drehvorrichtungen (70), wobei der Kreisbogen in zwei gebogene Teile unterteilt ist, die aneinander gleiten, und jeder der gebogenen Teile indirekt mit Stiften verbunden ist, die dazu bestimmt sind, beiderseits der Fraktur in jedes der Knochenfragmente eingeführt werden, **gekennzeichnet durch** zwei drehbare Platten (70, 80), in einem Winkel von 90° angeordnet und mit einem abgewinkelten Arm (60) verbunden, wobei eine der drehbaren Platten (70) über lineare Verstellvorrichtungen (10,20, 30) mit den Stiften an der einen Frakturseite verbunden und angeordnet ist, um ein linear graduelles Verstellen in drei Achsen zu ermöglichen, die andere drehbare Platten (80) mit dem einstellbaren Kreisbogen (90, 100) verbunden ist, an dem die Stifte der anderen Frakturseite befestigt sind, und jede drehbare Platte Vorrichtungen zum graduellen Verstellen in Drehbewegung aufweist.

2. Knochenrepositionsgerät nach Anspruch 1, **dadurch gekennzeichnet, dass** der abgewinkelte Arm aus einem für Röntgenstrahlen durchlässigen Material ist.

3. Knochenrepositionsgerät nach Anspruch 1, **dadurch gekennzeichnet, dass** die Achsen der Drehplatten eine Ebene definieren, auf der weitgehend die Fraktur liegt, und dass die Achse des einstellbaren Kreisbogens weitgehend rechtwinklig zu der von den Achsen der Drehplatten definierten Ebene liegt.

4. Knochenrepositionsgerät nach Anspruch 3, **dadurch gekennzeichnet, dass** jede Drehplatte (70, 80) einen Anker (71) enthält, mit einer Achse (72) verbunden, wobei die besagte Platte dazu dient, um die besagte Achse zu drehen und der Anker einen gezahnten Ausschnitt (73) aufweist, der in Zusammenarbeit mit einer Schnecke (74) zum graduellen Verstellen in Drehbewegung bestimmt ist.

5. Knochenrepositionsgerät nach Anspruch 4, **dadurch gekennzeichnet, dass** die Drehplatten in weitgehend rechtwinkligen Ebenen angeordnet sind und eine Drehbewegung von mehr oder weniger 30° in bezug auf eine Mittelposition zulassen.

6. Knochenrepositionsgerät nach Anspruch 1, **dadurch gekennzeichnet, dass** die linearen Verstellvorrichtungen drei Teleskopstangen (10, 20, 30) aufweisen, miteinander über Verbindungsteile (40, 50) verbunden, die in bezug auf die besagten Stangen beweglich sind.

7. Knochenrepositionsgerät nach Anspruch 6, **dadurch gekennzeichnet, dass** jedes Verbindungsteil (40, 50) mindestens eine Aussparung (41, 51, 56) aufweist, mit einer dem Querschnitt einer Stange entsprechenden Form, und die eine Dehnungsfuge (42, 52, 57) übergeht, sowie Feststellvorrichtungen (44, 54, 59), die sich zum Befestigen des Verbindungsteils an der entsprechenden Stange eignen.

8. Knochenrepositionsgerät nach Anspruch 6, **dadurch gekennzeichnet, dass** jede Teleskopstange (10, 20, 30) aus einem Innenrohr (11, 21, 31) gebildet wird, das mit einem Millimetermass (13, 23, 33) versehen ist, und einem Aussenrohr (12, 22, 32), das Pfeilangaben (14, 24, 34) aufweist, die sich zur Anzeige der Versetzung der Knochenfragmente nach den Drehrichtungen der Griffe (15, 25, 35) eignen, die mit Schnecken zum Verstellen der besagten Rohre verbunden sind.

9. Knochenrepositionsgerät nach Anspruch 8, **dadurch gekennzeichnet, dass** der mögliche Umfang der besagten Verstellung beiderseits einer Mittelposition einige Zentimeter beträgt.

10. Knochenrepositionsgerät nach Anspruch 6, **dadurch gekennzeichnet, dass** die besagten Stangen einen weitgehend dreieckigen Querschnitt haben.

11. Knochenrepositionsgerät nach Anspruch 6, **dadurch gekennzeichnet, dass** die besagten Stangen nach drei orthonormierten Achsen angeordnet sind.

12. Knochenrepositionsgerät nach Anspruch 6, **dadurch gekennzeichnet, dass** das Ende (27) einer der besagten Stangen eine Halterung für einen Schraubstock (6) zur Verbindung mit den Knochenstiften (4, Fig. 2) bildet.

13. Knochenrepositionsgerät nach Anspruch 1, **dadurch gekennzeichnet, dass** der einstellbare Kreisausschnitt von einem Bogen (90) gebildet wird, der mit den Verstellvorrichtungen eines beweglichen Wagens (100) verbunden ist.

14. Knochenrepositionsgerät nach Anspruch 13, **dadurch gekennzeichnet, dass** die besagten Verstellvorrichtungen aus einem gezahnten Kreisausschnitt (95) gebildet werden, der mit dem Bogen (90) verbunden ist und zur Zusammenarbeit mit einer Schnecke (104) dient, die mit dem beweglichen Wagen (100) verbunden ist.

15. Knochenrepositionsgerät nach Anspruch 13, **dadurch gekennzeichnet, dass** der Bogen eine gebogene Aussparung (93) aufweist, die zur Zusammenarbeit mit einer Gleitführung (101) dient, die mit dem beweglichen Wagen verbunden ist.

16. Knochenrepositionsgerät nach Anspruch 13, **dadurch gekennzeichnet, dass** der Bogen Gradeinteilungen (94) aufweist, die die Winkelversetzung des beweglichen Wagens in bezug auf den Bogen angeben.
